# EUROPEAN PATENT SPECIFICATION

(11) **EP 1 353 939 B1**
(45) Date of publication and mention of the grant of the patent: **25.08.2004**
(21) Application number: 01273785.4
(22) Date of filing: 16.10.2001
(51) Int. Cl.: C07K 5/11, A61K 38/07, A61P 13/08

(54) **TETRAPEPTIDE REGULATING PROSTATE FUNCTIONS AND ITS COMPOSITIONS AND USES**
TETRAPEPTID, DAS PROSTATAFUNKTIONEN REGULIERT SOWIE SEINE ZUSAMMENSETZUNGEN UND VERWENDUNGEN
TETRAPEPTIDE REGULANT LES FONCTIONS DE LA PROSTATE, SUBSTANCE PHARMACOLOGIQUE A BASE DE CE TETRAPEPTIDE ET SES APPLICATIONS

(30) Priority: 25.01.2001 RU 2001102099
(43) Date of publication of application: 22.10.2003
(73) Proprietor: Sankt-Peterburgskaya Obschestvennaya Organizatsia "Sankt-Peterburgskiy Institut Bioregulyatsii I Gerontologii Szo Ramn", St. Petersburg 197110 (RU)
(72) Inventor: KHAVINSON, Vladimir Khatskelevich, St.Petersburg, 197374 (RU); MALININ, Vladimir Viktorovich, St.Peterburg, 197227 (RU); GRIGORIEV, Evgeny Iosifovich, St.Petersburg, 192284 (RU)
(74) Representative: Läufer, Martina, Dr.
(86) International application number: PCT/RU2001/000433
(87) International publication number: WO 2002/066497

(56) References cited:
- PETRISHCHEV NN ET AL.: "The effect of a polypeptide complex isolated from the animal prostate on thrombus formation" PATOLOGICHESKAYA FIZIOLOGIYA I EKSPERIMENTAL'NAYA TERAPIYA, no. 4, 1991, pages 5-6, XP008006298
- SUSSET JG ET AL.: "The effect of raveron on the detrusor muscle" JOURNAL OF UROLOGY, vol. 141, no. 2, February 1989 (1989-02), pages 408-413, XP008006299

### SEQUENCE LISTING

<110> SANKT-PETERBURGSKAYA OBSCHESTVENNAYA ORGANIZATSIYA "SANKT-PETERBURGSKII INSTITUT BIOREGULYATSII I GERONTOLOGII SZO RAMN"
<120> Tetrapeptide regulating prostate functions, pharmacological substance on its basis and method of its application
<130> IBG/07/01
<140>
   <141>
<150> RU 2001102099
   <151> 2001-01-25
<160> 1
<170> PatentIn Ver. 2.1
<210> 1
   <211> 4
   <212> PRT
   <213> Artificial Sequence
<220>
   <223> Tetrapeptide Lys Glu Asp Pro regulating the prostate functions
<400> 1
   Lys Glu Asp Pro
     1

## Description

The invention refers to the field of medicine and may be applied as a substance regulating the prostate functions in prevention of and treatment for diseases of the prostate.

Diseases of the prostate gland belong to the most serious and consequential pathologies in the contemporary urology. Their incidence tends to increase and the social significance grows ever faster (1,2).

Conservative treatment for prostate diseases chiefly employs - with respect to their pathogenetic mechanisms - the following traditional therapeutic substances of various effects (3):
- antibiotics and sulphanilamide medications - to exert anti-bacterial and anti-inflammatory effects;
- Trental, Halidor, Aescusan - to normalise microcirculation;
- No-Spa, Baralgine - to exert a spasmolytic effect;
- methyl testosterone, Sustanon-250 - to normalise the level of sex hormones;
- Prodigiosan, Pyrogenal, Levamisol, Tavegil, Phencarol - to correct immunopathologic reactions;

However, application of these pharmaceuticals enables to affect only separate aetiopathogenetic aspects of different prostate diseases and exerts no specific effect upon the prostate tissue.

The proposed peptide compound is a tetrapeptide having no structural analogues.

Among the closest application analogues of this preparation, there are known pharmaceuticals stimulating metabolic processes in the prostate gland: Raveron (3) and Prostatilen (4).

Prostatilen is chosen as the prototype for the proposed pharmaceutical preparation. It is a polypeptide preparation obtained from the prostate tissue of puberal animals. The sphere of its application is however restricted due to the complicated technology of obtaining it (5), small outcome of active substances, high variability of its physico-chemical properties and possible allergenic effects.

Prostatilen is administered to patients parenterally in courses until attaining a therapeutic effect.

The proposed invention is designed to obtain a biologically active compound of peptide nature regulating the prostate functions.

The present invention describes tetrapeptide lysyl-glutamyl-aspartyl-proline of the general formula Lys-Glu-Asp-Pro. Tetrapeptide lysyl-glutamyl-aspartyl-proline of the amino acid sequence Lys-GIu-Asp-Pro reveals biological activity, and namely, restores the functions of the prostate and urinary bladder wall, normalises the regional immunity indices, stimulates anti-inflammatory reactions and parameters of the anti-oxidation defence system.

The tetrapeptide is obtained by a classical method of peptide synthesis in a solution (6).

The regulatory effect of Lys-Glu-Asp-Pro tetrapeptide on the prostate functions has been revealed in experiment.

Biological activity of the tetrapeptide has been studied in urinary bladder explants - to investigate its tissue-specific activity, in rats - to study its anti-inflammatory effect in case of bacterial prostatitis and anti-oxidation defence system indices in senescent animals.

The pharmaceutical preparation of the present invention regulating the prostate functions contains as its active base an effective amount of tetrapeptide of the formula lysyl-glutamyl-aspartyl-proline (Lys-Glu-Asp-Pro) or one of its salts.

The pharmaceutical preparation of the present invention regulating the prostate functions may contain salts of the amino group (acetate, hydrochloride, oxalate) or of carboxyl groups (salts of metals - sodium, potassium, calcium, lithium, zinc, magnesium, as well as of other organic and inorganic cations - ammonium and triethylammonium).

The pharmaceutical preparation of the present invention is intended for parenteral administration.

The proposed pharmaceutical preparation regulates the prostate functions, produces anti-inflammatory and anti-oxidative effects.

The notion "pharmaceutical preparation" of the present invention implies the use of any drug form containing the tetrapeptide or one of its salts, which can find its preventive and/or therapeutic employment in medicine as a preparation regulating the prostate functions.

The notion "effective amount" of the present invention implies the use of such an amount of the active base, which, in compliance with the quantitative indices of its activity and toxicity, as well as with respect to the knowledge available, shall be effective in this drug form.

To obtain pharmaceutical preparation meeting the invention, the proposed tetrapeptide or its pharmaceutically applicable derivatives in the form of salts are blended as an active ingredient and a pharmaceutical carrier in accordance with the methods of compounding accepted in pharmaceutics. The carrier may have various forms depending on the drug form of the preparation desirable for introduction into a body, for example parenteral administration.

The carrier for parenteral administration usually includes sterile water, though there could be employed other ingredients instrumental for stability or maintaining sterility.

Further disclosed is the use of the tetrapeptide of the present invention for the preparation of a medicament for regulating the prostate functions by exerting anti-inflammatory and anti-oxidative effects by administering to a patient the proposed pharmaceutical preparation in the doses of 0.01-100 µg/kg of the body weight at least once a day during a period required for attaining a therapeutic effect, i.e. 10-40 days with respect to the character and severity of the treated pathological process.

The pharmaceutical preparation of the present invention is active when administered in the doses of 0.01-100 µg/kg of the body weight, though low/higher doses are admissible depending on the character and severity of the treated pathologic process.

The invention is illustrated by an example of synthesis of tetrapeptide lysyl-glutamyl-aspartyl-proline (Lys-Glu-Asp-Pro) (Example 1), examples of testing the tetrapeptide for toxicity and biological activity (Examples 2, 3, 4, 5) and examples of the clinical application of the pharmaceutical preparation demonstrating its pharmacological properties and confirming the possibility of attaining a preventive and/or therapeutic effect (Example 6, 7).

### Example 1. Synthesis of Lys-Glu-Asp-Pro tetrapeptide

1. *Product name:* **lysyl-glutamyl-aspartyl-proline.**
2. Structural formula: 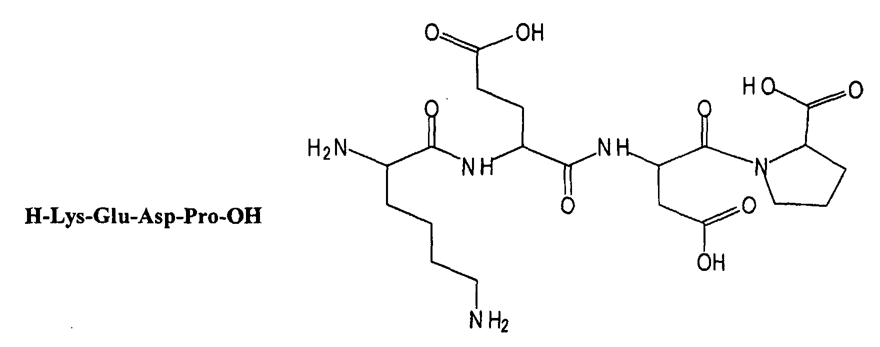
3. Molecular formula without ion pair: C₂₀H₃₃N₅O₉.
4. Molecular weight without ion pair: 487.51.
5. Ion pair: acetate.
6. Appearance: white amorphous powder without smell.
7. Method of synthesis: the peptide is obtained by a classical method of synthesis in a solution by the following scheme:

N,N'-dimethylformamide was used as a solvent. When adding aspartic acid, the defence of α-COOH group was applied by salification with triethylamine. BOC-protecting group was removed with trifluoracetic acid (TFA) solution and Z-protecting groups - with catalytic hydrogenation. The product was extracted and purified by the method of preparative high-performance liquid chromatography (HPLC) on a reversed phase column.

Properties of the finished product:
• amino acid analysis

| **Lys** | **Glu** | **Asp** | **Pro** |
|---|---|---|---|
| **0.98** | **1.01** | **1.01** | **1.00** |

• peptide content 98.56 % (by HPLC, 220 nm);
• thin layer chromatography (TLC) - individual, R_{f}=0.61 (Silicagel, acetonitrile-water 1:1);
• moisture content: 6 %;
• pH of 0.001 %-solution: 5.55;
• specific rotary power: [α]_{D}²³: -69.2° (c=1.04; H₂O), "Polamat A", Carl Zeiβ Jena.

### Example of synthesis:

### 1. BOC-Glu(OBzl)-Asp(OBzl)-OH(I), N-tert.butyloxycarbonyl-(γ-benzyl)glutamyl-(β-benzyl)aspartate.

4.34 g (0.0100 mol) of N-oxysuccinimide ester of N-tert.butyloxycarbonyl-(γ-benzyl)glutamic acid (BOC-Glu(OBzl)-OSu) is dissolved in 20 ml of dimethylformamide and added 1.72 ml (0.0125 mol) of triethylamine and 2.80 g (0.0125 mol) of β-benzyl aspartate. The mixture is stirred for 24 hours at room temperature. Afterwards the product is precipitated with 0.5N sulphuric acid solution (150 ml), extracted by ethyl acetate (3x30 ml), washed in 0.5N sulphuric acid solution (2x20 ml), water, 5 % sodium bicarbonate solution (1x20 ml), water, 0.5N sulphuric acid solution (2x20 ml), water. The product is dried over anhydrous Na₂SO₄. Ethyl acetate is filtered and removed *in vacuo* at 40°C, the residue is dried *in vacuo* over P₂O₅. 5.68 g (≈100 %) of oil is obtained. R_{f}=0.42 (benzene-acetone 2:1, Sorbfil plates,

Silicagel - 8-12 µm, development by UV and chlorine/benzidine).

### 2. TFAH-Glu(OBzl)-Asp(OBzl)-OH (II), (γ-benzyl)glutamyl-(β-benzyl) aspartate trifluoracetate

5.68 g (≈0.01 mole) of N-tert.butyloxycarbonyl-(γ-benzyl)glutamyl-(β-benzyl)aspartate (I) is dissolved in 20 ml of dichlormethan-trifluoracetic acid mixture (3:1). Two hours later the solvent is removed *in vacuo* at 40°C. The removal is repeated with an addition of another portion of dichlormethan (2x10 ml). The residue is dried *in vacuo* over NaOH. 5.80 g (≈100 %) of oil is obtained. R_{f}=0.63 (n-butanol-pyridine-acetic acid-water, 15:10:3:12).

### 3. Z-Lys(Z)-Glu(OBzl)-Asp(OBzl)-OH (III), N,N^{ε}-dibenzyloxycarbonyllysyl-(γ-benzyl)glutamyl-(β-benzyl)aspartate.

5.65 g (0.01 mol) of (γ-benzyl)glutamyl-(β-benzyl)aspartate trifluoracetate (II) is dissolved in 10 ml of dimethylformamide, added 2.80 ml (0.02 mol) of triethylamine and 6.64 g (0.013 mol) of N-oxysuccinimide ester of N,N^{ε}-dibenzyloxycarbonyllysine. The reacting mixture is stirred for 24 hours at room temperature.

The product is precipitated with 0.5N sulphuric acid solution (150 ml), extracted by ethyl acetate (3x30 ml), washed in 0.5N sulphuric acid solution (2x20 ml), water, 5 % sodium bicarbonate solution (1x20 ml), water, 0.5N sulphuric acid solution (2x20 ml), water and dried over anhydrous sodium sulphate. Ethyl acetate is filtered and removed *in vacuo* at 40°C. The residue is recrystallised in the ethyl acetate/hexane system. The product is filtered and dried *in vacuo* over P₂O₅. The yield is 6.04 g (72 %). The temperature of melting (Tₘₗ) is 142°C. R_{f}=0.60 (benzene-acetone, 1:1).

### 4. Z-Lys(Z)-Glu(OBzl)-Asp(OBzl)-Pro-OBzl (IV), N,N^{ε}-dibenzyloxycarbonyllysyl-(γ-benzyl)glutamyl-(β-benzyl)aspartyl-proline benzyl ester.

0.72 g (3 mmol) of chloride of proline benzyl ester (HCl H-Pro-OBzl) is suspended in 15 ml of tetrahydrofuran and added 0.4 ml (3 mmol) of triethylamine while stirring. In 5 minutes 1.68 g (2 mmole) of N,N^{ε}-dibenzyloxycarbonyllysyl-(γ-benzyl)glutamyl-(β-benzyl)aspartate (III) and 0.27 g (2 mmol) of N-oxybenzotriazol are added. The mixture is cooled down to 0°C. Afterwards, 0.42 g (2 mmol) of N,N'-dicyclohexylcarbodiimide solution cooled down to 0°C is added in 5 ml of tetrahydrofuran. The mixture is stirred at this temperature for 2 hours and left to blend for a night at room temperature. The residue of dicyclohexylurea is filtered out, the solvent is removed *in vacuo* and the residue is dissolved in 30 ml of ethyl acetate. The solution is washed in 1N sulphuric acid solution, water, 5 % sodium bicarbonate solution, water, 1N sulphuric acid solution, water and dried over anhydrous sodium sulphate. The solvent is removed *in vacuo* and the product is recrystallised in the ethyl acetate/hexane system. The yield is 1.39 g (68 %). Tₘₗ=147-150°C. R_{f}=0.54 (benzene-acetone, 2:1).

### 5. H-Lys-Glu-Asp-Pro-OH (V), lysyl-glutamyl-aspartyl-proline.

1.24 g of benzyl ester of N,N^{ε}-dibenzyloxycarbonyllysyl-(γ-benzyl)glutamyl-(β-benzyl)aspartyl-proline (III) is hydrogenated in the methanol/water/acetic acid system (3:1:1) over Pd/C catalyst. Completeness of the deblocking reaction is monitored by TLC method in the benzene/acetone (2:1) and acetonitrile/acetic acid/water (5:1:3) systems. At the reaction completion the catalyst is filtered out, the filtrate is removed *in vacuo* and the residue is recrystallised in the water/methanol system. The product is dried *in vacuo* over KOH. The yield is 0.526 g (90 %). R_{f}=0.61 (acetonitrile/water, 1:1). For purification, 500 mg of the product is dissolved in 4 ml of 0.0 1 % trifluoracetic acid (sample pH is 2.21) and subjected to HPLC on a reversed phase column (50x250 mm, Diasorb-130-C16T, 7 µ). The employed chromatograph is Beckman System Gold, 126 Solvent Module, 168 Diode Array Detector Module. The conditions of chromatography A: 0.1 % of TFA; B: 50 % of MeCN/0.1 % of TFA, gradient B 0 → 16 % in 240 minutes. Sample volume constitutes 5 ml, detection is conducted by 215 nm, scanning - by 190-600 nm. The flow rate equals 10 ml/min. The fraction is selected within 105-135 min.
The solvent is removed *in vacuo* at a temperature not exceeding 40°C. The removal is multiply repeated (5 times) with 10 ml of 10 % acetic acid solution. The residue is finally dissolved in 20 ml of deionised water and lyophilised. 280 mg of purified product in the form of amorphous odourless white powder is obtained.
In order to obtain corresponding salts of carboxyl groups, the free tetrapeptide is added a calculated amount of the aqueous solution of a corresponding metal hydroxide (NaOH, KOH, Zn(OH)₂, LiOH, Ca(OH)₂, Mg(OH)₂, NH₄OH). To obtain the salt of triethylammonium, the processing is carried out similarly, triethylamine being used as the base.

### 6. Analysis of the finished product.

• Peptide content is defined by HPLC on Supelco LC-18-DB column, 4.6x250 mm, grad. LC-18-DB. A: 0.1 % of TFA; B: 50 % of MeCN/0.1 % of TFA; grad. B 0 → 20 % in 30 min. The flow speed equals 1 ml/min. Detection by 220 nm, scanning - by 190-600 nm, the sample volume is 20 µl. Peptide content - 98.56 %.
• Amino acid analysis is carried out on AAA "T-339" tester, Prague. Hydrolysis is conducted in 6N HCl at 125°C for 24 hours.

| **Lys** | **Glu** | **Asp** | **Pro** |
|---|---|---|---|
| 0.98 | 1.01 | 1.01 | 1.00 |

• TLC: individual, R_{f}=0.61 (acetonitrile/water, 1:1). Sorbfil plates, 8-12 µm Silicagel, developing in chlorine/benzidine.
• Moisture content: 6 % (gravimetrically, according to the mass loss by drying, - 20 mg at 100°C).
• pH of 0.001 % solution: 5.55 (potentiometrically)
• Specific rotary power: [α]_{D}²³: -69.2° (c=1.04, H₂O) "Polamat A", Carl Zeiβ Jena.

The pharmaceutical preparation in the form of solution is obtained the following way: the tetrapeptide obtained by the above-described method is dissolved in 0.9 % isotonic natural saline solution. The concentration of the tetrapeptide equals 10 µg per 1 ml of natural saline solution.

### Example 2. Study of Lys-Glu-Asp-Pro tetrapeptide for toxicity

The possible toxic effect of Lys-Glu-Asp-Pro tetrapeptide on the human organism was studied in compliance with "The Rules of Pre-Clinical Assessment of the Safety of Pharmacological Substances" (GLP).

This study was designed to define the tolerable toxic doses of Lys-Glu-Asp-Pro tetrapeptide, assess the degree and character of pathologic alterations in various organs and systems of the organism and reveal the dependence of toxic effects on the dosage and duration of the intake of the tetrapeptide.

Acute toxicity of Lys-Glu-Asp-Pro tetrapeptide was defined according to Kerber. Examined were 78 white mongrel male mice weighing 20-22 g. The mice were kept in *vivarium* under standard regimen and fed upon standard rations. They were randomised to six equal groups, 13 mice in each. The animals were intramuscularly injected with the tetrapeptide once in the doses of 1 mg/kg, 2 mg/kg, 3 mg/ kg, 4 mg/kg and 5 mg/kg 0.25 ml of natural saline solution (several thousand times exceeding the therapeutic dose recommended for clinical trials). The control animals received the same amount of natural saline solution.

During 72 hours and later in 14 days none of the animals died in either of the groups. No alterations in their general state, behaviour, motor activity, hair and skin integument, physiological discharge were registered.

Consequently, Lys-Glu-Asp-Pro tetrapeptide administered in doses, which several thousand times exceed the therapeutic one recommended for clinical trials, does not entail any acute toxic reactions, thus revealing a wide therapeutic applicability of the preparation.

Subacute toxicity of Lys-Glu-Asp-Pro tetrapeptide was investigated on 48 white mongrel rats weighing 170-240 g. The animals were injected with the tetrapeptide intramuscularly in the doses of 1 µg/kg, 0.3 mg/kg, 3 mg/kg in 0.5 ml of natural saline solution, once a day for 90 days. The control animals were administered with natural saline solution in the same amount.

The animals were observed daily for the whole course of investigation. Their behaviour, food and water consumption, state of hair integument and mucous membranes were noted. The rats were weighed weekly. Before the administration of the tetrapeptide and on the 30^{th}, 60^{th} and 90^{th} days thereof, the morphological composition and properties of their peripheral blood were examined. Biochemical and coagulological properties of the blood were studied at the experiment completion.

The tetrapeptide was investigated for chronic toxicity on 56 white mongrel rats weighing 150-270 g. The animals were intramuscularly injected with the tetrapeptide in the doses of 1 µg/kg, 0.1 mg/kg, 1 mg/kg in 0.5 ml of natural saline solution, once a day for 6 months.

Their behaviour, food and water consumption, state of hair integument and mucous membranes were examined. The rats were weighed daily during the first three months of the experiment and then once a month. In three months after the administration onset and at the experiment completion, haematological and biochemical investigations were conducted. The functions of the animals' cardiovascular system, liver, pancreas, kidneys and adrenal glands were assessed. At the end of administering the tetrapeptide, part of the animals were subjected to a *post mortem* examination to assess the state of various segments of their brain and spinal marrow, heart, aorta, lungs, liver, kidneys, endocrine and immune organs.

Assessment of the animals' general state, morphological and biochemical indices of their peripheral blood, morphological state of their intrinsic organs, cardiovascular and respiratory systems, liver and kidney functions revealed no pathologic alterations.

Study of Lys-Glu-Asp-Pro tetrapeptide for subacute and chronic toxicity demonstrates the lack of any side effects in case of long-term application of the tetrapeptide in doses, which exceed the therapeutic one 100-1000 times.

### Example 3. Effect of Lys-Glu-Asp-Pro tetrapeptide on the development of urinary bladder explants

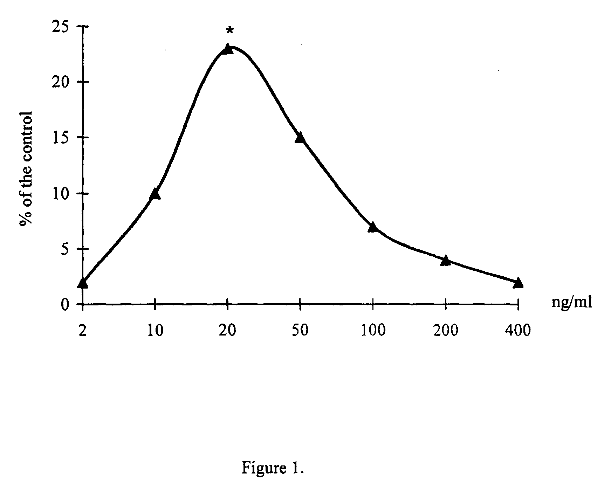

The experiments were carried out in 45 urinary bladder fragments of 10-11-days old chicken embryos. The nutrient medium for the explant cultivation included 35 % of Eagle's solution, 25 % of foetal calf serum, 35 % of Hanks' solution, 5 % of chicken embryonic extract and was added glucose (0.6 %), insulin (0.5 unit/ml), penicillin (100 unit/ml) and glutamine (2 mM). The urinary bladder fragments were placed in this medium and cultivated in Petri's dishes in a thermostat at 36.7°C for 48 hours. Lys-Glu-Asp-Pro tetrapeptide was added to the experimental medium in the concentrations of 2, 10, 20, 50, 100, 200 and 400 ng/ml. Square index (SI) was taken for a biological activity criterion and calculated as a correlation of the total explant square (including the growth zone) to the initial square of a urinary bladder fragment. The SI values were expressed per cent, the control SI value - taken for 100 %. Figure 1 demonstrates the effect of Lys-Glu-Asp-Pro tetrapeptide on the development of the urinary bladder explants (*p<0.05 in comparison with the control indices taken for 100 %).

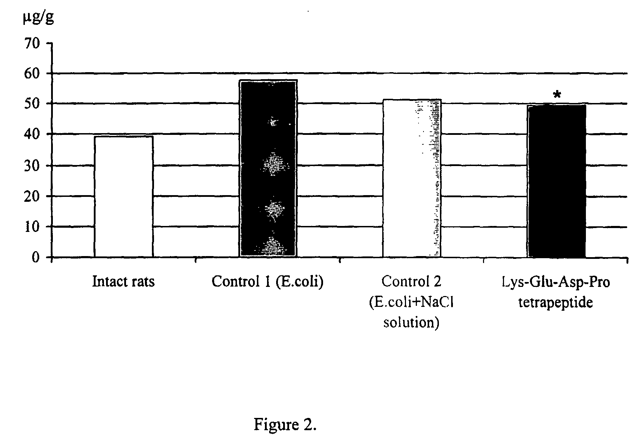

After 24 hours of cultivation, the explants on a collagen lining were found to lie flat. Proliferating and migrating cells started to move along the explant periphery. By the concentration of Lys-Glu-Asp-Pro equalling 20 ng/ml, a significant increase in the explant SI was observed on the third day of cultivation (by 23 % as compared to the control value) (Figure 2). Figure 2 exhibits the effect of Lys-Glu-Asp-Pro tetrapeptide on zinc concentration in the prostate tissue of rats with chronic bacterial prostatitis (*p<0.05 in comparison with the indices for the 1^{st} control group). In case of longer terms of the liver explant cultivation (up to 7 days), an analogous stimulating effect of the preparation in the same concentration was revealed.

Consequently, Lys-Glu-Asp-Pro tetrapeptide exerts a stimulating regulatory effect upon urinary bladder explant growth.

### Example 4. Effect of Lys-Glu-Asp-Pro tetrapeptide on the course of experimental chronic prostatitis

The investigation was performed on 41 white mongrel male rats weighing on average 230 g. Bacterial prostatitis was induced in the rats by transurethral introduction of enterotoxigenic E.coli suspension K-2935 (01: K1: H-). A glycerine-lubricated catheter was inserted into the animals' urethra under a light ether narcosis after treating the external urethra opening with 70 % alcohol. 0.2 ml of bacterial suspension (10⁹ colony-forming units per ml) was instilled via catheter with a syringe. Control animals were injected with 0.2 ml of 0.9 % natural saline solution through the catheter. In 45 days after the bacterial infection, the rats with developed chronic prostatitis began to receive Lys-Glu-Asp-Pro tetrapeptide subcutaneously in the dose of 0.1 µg per injection. The injections were performed daily for 10 days. The control groups included intact animals, animals with untreated chronic prostatitis and those treated with injections of 0.9 % natural saline solution applied by the same plan.

All the rats were euthanasised by decapitation on the 70^{th} day of the experiment. The animals were subjected to complete autopsy. Their testes and epididymes, prostate, seminal vesicles, bulbourethral glands, urinary bladder, kidneys with ureters and other intrinsic organs were examined. For histology the ventral segment of the prostate was fixed in 10 % neutral formalin and - after a standard processing with a series of solvents - embedded in paraffin. 5-7-µm thin tissue sections were stained with haematoxylin-eosin.

The degree of prostatitis manifestation throughout the groups was assessed according to the below 5-point scale:
1 point - traces of prostatitis (small foci of fibrosis, interstitial lymphocytic and histiocytic infiltration);
2 points - foci of chronic prostatitis with inflammatory infiltration (basically, interstitial one) occupying less than ¼ of the gland section;
3 points - prostatitis foci with large inflammatory infiltrates surrounding the ducts, acini and blood vessels, with leukocytic conglomerations in the glandular lumens, embracing ¼ - ½ of the gland section;
4 points - expressed prostatitis covering ½ - ¾ of the gland section;
5 points - strongly expressed prostatitis affecting over ¾ of the gland section.

The prostate sites were also used for electron microscopic examination and tests on the content of zinc, which makes a part of compositions with anti-microbial activity.

The conducted study revealed that application of Lys-Glu-Asp-Pro tetrapeptide promoted a positive dynamics of inflammatory processes in the prostate in 7 cases out of 11. Histological examination of the prostate sections mostly exposed the signs of chronic inflammation covering less than ½ of the gland section. Thereby, inflammatory infiltrates were not observed in the majority of the ducts and acini. The average point of prostatitis degree equalled 1.81 ± 0.09, as demonstrated in Table 1.

**Table 1**

| *Characteristics of the degree of chronic bacterial prostatitis in rats* | | | | |
|---|---|---|---|---|
| Group of animals | Number of rats in the group | | Degree of prostatitis in points | |
| | Total | Prostatitis -affected | Per rat in the group | Per prostatitis-affected rat |
| Intact rats | 8 | - | - | - |
| Control 1 (E.coli) | 11 | 8 | 2.91 ±0.15 | 4.0 |
| Control 2 (E.coli + natural saline solution) | 11 | 7 | 2.36±0.12 | 3.71 |
| E.coli + Lys-Glu-Asp-Pro tetrapeptide | 11 | 7 | 1.81 ± 0.09* | 2.86* |

| | | | | |
|---|---|---|---|---|
| * - P<0.05 in comparison with the indices for the 1^{st} and 2^{nd} control groups. | | | | |

Zinc content in the prostate tissue increased depending on the degree of prostatitis development. After the application of the tetrapeptide the mean concentration of zinc decreased (Figure 2), which correlated with the degree of prostatitis manifestation.

Thus, application of Lys-Glu-Asp-Pro tetrapeptide activates the reactions of regional immunity and raises the anti-microbial defence of the prostate tissue accompanied by decreased inflammatory reactions.

### Example 5. Effect of Lys-Glu Asp-Pro tetrapeptide on the processes of free radical oxidation in senescent rats

Disorders of anti-oxidation defence and neurohumoral regulation of the organism functions accompanied, among other, by sex gland involution, refer to the most typical signs of ageing.

The effect of Lys-Glu-Asp-Pro tetrapeptide upon the processes of free radical oxidation was investigated in 18 male rats aged 24 months. The animals were subcutaneously administered with 0.1 µg of the tetrapeptide per injection for 10 days. The controls were similarly injected with 0.9 % natural saline solution. The rats were euthanasised on the next day after the last introduction of the tetrapeptide.

Afterwards, samples of the brain and liver tissues and blood serum were prepared and peroxide lipid oxidation (PLO) intensity in them was studied by the content of diene conjugates and Schiff's bases. The activity of glutathione peroxidase and superoxide dismutase was defined as well by the method of suppressing nitroblue tetrasolium oxidation with biological material.

In the study Lys-Glu-Asp-Pro tetrapeptide was found to facilitate a decrease in the formation of diene conjugates - PLO start products - in the liver and Schiff bases - PLO end products - in the blood serum.

Besides, Lys-Glu-Asp-Pro tetrapeptide exerted a significant effect upon the ferment link of the anti-oxidation system in the liver and brain. Lys-Glu-Asp-Pro increased glutathione peroxidase activity in the liver and superoxide dismutase one - in the brain.

Consequently, application of Lys-Glu-Asp-Pro tetrapeptide in senescent male rats normalises the mechanisms of anti-oxidation defence associated with age-related involutionary changes in their reproductive organs.

The experimental study proved the tetrapeptide to be toxic-free, to stimulate urinary bladder explant growth, facilitate activation of the regional immunity reactions, normalise metabolic and microcirculation processes in the prostate and to possess an anti-oxidative activity.

Properties of Lys-Glu-Asp-Pro tetrapeptide revealed in its experimental pre-clinical study substantiate the preventive and/or therapeutic applicability of the drug as a pharmaceutical in the treatment for the prostate diseases accompanied by inflammatory disorders and dysury.

**Table 2**

| *Effect of Lys-Glu-Asp-Pro tetrapeptide upon the anti- and pro-oxidation defence indices in the blood serum, brain and liver of senescent rats* | | |
|---|---|---|
| Indices | Control | Lys-Glu-Asp-Pro tetrapeptide |
| Blood serum | | |
| Diene conjugates (nmol/ml) | 5.54 ± 0.34 | 5.28 ± 0.33 |
| Schiff's bases (conv. unit/ml) | 25.20 ± 1.74 | 17.40 ± 1.54* |
| Glutathione peroxidase (mmol of GlSH/min/mg of protein) | 2.82 ± 0.06 | 2.85 ± 0.06 |
| Superoxide dismutase (conv. unit/mg of protein) | 0.854± 0.037 | 0.826 ± 0.060 |

| Brain | | |
|---|---|---|
| Diene conjugates (mnol/ml) | 44.89 ± 0.88 | 46.14 ± 2.91 |
| Schiff's bases (conv. unit/ml) | 274.8 ± 13.7 | 237.3 ± 17.8 |
| Glutathione peroxidase (mmole of GlSH/min/mg of protein) | 48.9 ± 1.8 | 50.7±0.2* |
| Superoxide dismutase (conv. unit/mg of protein) | 24.8 ± 2.8 | 26.7 ± 3.0 |

| Liver | | |
|---|---|---|
| Diene conjugates (nmol/ml) | 53.19 ± 2.16 | 44.76 ± 2.48* |
| Schiff's bases (conv. unit/ml) | 661.3 ± 31.1 | 556.5 ±37.6 |
| Glutathione peroxidase (mmol of GlSH/min/mg of protein) | 701.4 ± 17.1 | 798.0±29.1* |
| Superoxide dismutase (conv. unit/mg of protein) | 48.3 ± 6.4 | 54.1 ± 7.9 |

| | | |
|---|---|---|
| * - P<0.05 in comparison with the control indices. | | |

The below-adduced examples on the results of the clinical application of the proposed tetrapeptide demonstrate its pharmacological properties and confirm the possibility of achieving a therapeutic effect by introducing the pharmaceutical preparation containing Lys-Glu-Asp-Pro tetrapeptide.

### Example 6. Efficacy of applying the pharmaceutical preparation in the patients with chronic prostatitis

The efficacy of clinical application of the pharmaceutical preparation was studied in 35 patients aged 23-45 with chronic prostatitis. Chronic prostatitis was normally manifested in boring pain of typical location, diverse urination disorders, sexual dysfunction and neurotic signs.

All the patients had previously taken conventional pharmaceuticals of symptomatic and pathogenetic action, which exerted a short-term therapeutic effect and required their prolonged administration and ever-higher doses within one treatment course.

The pharmaceutical preparation was intramuscularly administered to the patients in the dose of 0.01-100 µg/kg of the body weight, once a day for 10-40 days depending on the disease severity. The control group embraced 14 similar patients treated by conventional methods.

The efficacy of treating patients with the pharmaceutical preparation was assessed basing on the patients' complaints, by total blood count, blood coagulogram, biochemical blood test and urinalysis before and after the treatment course.

The degree of abdominal pressure at urination and character of urine stream were expressed according to a 5-point scale (1 point - norm, 5 points - the topmost change in the index). The maximal and mean rate and time of urination, the time of attaining the maximal urination rate and fluorimetric index were estimated. Besides, the prostate was examined by palpation, its secretion - analysed in laboratory tests and the copulative function - studied more thoroughly. The prostate was also by investigated ultrasound.

Clinical criteria were in the major focus of attention in assessing the results of treating for chronic prostatitis. After the treatment 64.0 % of the patients registered complete disappearance of pain and 32.7 % - significantly decreased painful sensations. The therapy did not bring the expected results to 3.3 % of the patients who noted no dynamics in the pain syndrome. 44.4 % of the patients with disturbed sexual function noted its full recovery and 41.8 % - its improvement.

Prolonged effect of the pharmaceutical preparation was manifested both in relatively improved quality of erection and in stronger and painless orgasm. The time of sexual act was also prolonged. By the end of the treatment course, 37.1 % of the patients reported of libido normalisation.

Pollakiuria (frequent urination) stopped disturbing 87.5 % of the patients completely. The imperative need for nocturnal urination disappeared. Strangury (difficult urination) ceased in 80.7 % of the cases, 15.9 % of the patients registered notable increase in the urine stream tension and easier urination.

Table 3 demonstrates the integral data on urination changes in chronic prostatitis patients after the conducted therapy with the pharmaceutical preparation.

**Table 3**

| *Effect of the pharmaceutical preparation on the urodynamics in chronic prostatitis patients* | | |
|---|---|---|
| Index | Before treatment | After the treatment with the pharmaceutical preparation |
| Mean urination rate (ml/sec) | 17.3 ± 1.2 | 23.5 ± 2.4* |
| Maximal urination rate (ml/sec) | 22.1 ± 2.3 | 26.5 ± 3.1 |
| Time of attaining the maximal urination rate (ml/sec) | 3.6 ± 0.2 | 1.5 ± 0.1* |

| | | |
|---|---|---|
| * - P<0.05 in comparison with the indices before treatment. | | |

It must be emphasised that uroflowgrams taken after treating the patients with chronic prostatitis of the first and second stages revealed restoration of the basic urination parameters up to the normal values.

At the third stage of the disease this was impeded by lowered elasticity of the urinary bladder neck due to sclerotic alterations of the prostate tissue, but even in such cases, a significant increase in the urine stream was noted. Palpation of the prostate via colon enabled to define a tendency to the restoration of the gland sizes and consistence after the therapy with the phannaceutical preparation. Thereby, consolidated sites disappeared and palpation became painless. Ultrasound diagnosis data confirmed the observed decrease in, the prostate size, which was estimated as a result of lessened interstitial tissue oedema and evidenced liquidation of the inflammatory process in the gland. Dynamically conducted total blood count and biochemical blood test did not reveal any significant deviations from the normal values.

The prostate functions restored under the effect of the pharmaceutical preparation resulted in improved properties of its secretion. This raised the content of active spemiatozoids in the ejaculate by 11.8 %. Reduced number of leukocytes in the ejaculate, prostate secretion and urine reflected the decreased activity of the inflammatory process.

At the same time, a decreased content of the peeled epithelium cells in the examined material was observed.

### Example 7. Efficacy of applying the pharmaceutical preparation in the patients with prostate adenoma

The pharmaceutical preparation was applied in 19 patients with prostate adenoma at Stages I-II (51-67 y. o.). Typical symptoms of prostate adenoma in the patients consisted in stable dysury incurable by the conventional methods, weak urine stream and decreased sexual ability. All the patients had been prior treated with conventional symptomatic and pathogenetic pharmaceuticals for different periods. The patients were intramuscularly injected with the pharmaceutical preparation in the dose of 0.01-100 µg/kg of the body weight once a day for 10-40 days depending on the clinical picture of the disease. The control group included 17 analogous patients treated by the conventional methods.

The efficacy of the therapy with the pharmaceutical preparation was evaluated basing on the dynamics of the patients' complaints. The degree of abdominal pressure at urination and character of urine stream were expressed according to a 5-point scale (1 point - norm, 5 points - the topmost change in the index). The maximal and mean rate and time of urination, the time of attaining the maximal urination rate and fluorimetric index were estimated. The prostate was studied by ultrasound. Table 4 exhibits the dynamics of the results obtained in the patients with prostate adenoma before and after a course of the therapy with the pharmaceutical preparation.

**Table 4**

| *Effect of the pharmaceutical preparation on the urodynamics in prostate adenoma patients* | | |
|---|---|---|
| Indices | Before treatment | After the treatment with the pharmaceutical preparation |
| Time of urination detention (min) | 4.6 ± 0.3 | 2.5 ± 0.1* |
| Number of urinations - in the daytime - at night | 8.4 ± 0.5 3.1 ±0.1 | 6.3 ± 0. 1* 2.6 ± 0.1 |
| Degree of abdominal pressure (points) | 3.2 | 2.4 |
| Character of the urine stream (points) | 3.3 | 2.3* |
| Mean urination rate (ml/sec) | 11.3 ± 1.2 | 17.5 ± 1.6* |
| Maximal urination rate (ml/sec) | 16.1 ± 2.3 | 20.5 ± 3.1 |
| Time of attaining the maximal urination rate (ml/sec) | 6.6 ±0.2 | 4.5 ±0.1* |

| | | |
|---|---|---|
| * - P<0.05 in comparison with the indices before treatment. | | |

State of the patients with prostate adenoma after the treatment with the pharmaceutical preparation was accompanied by better subjective and objective urodynamics indices. 37.3 % of the patients noted an increase in libido by the end of the treatment course.

Thus, the pharmaceutical preparation is expedient to be applied in the treatment for inflammatory processes of the prostate and dysuretic disorders.

### References

1. Lyulko A.V., Yunda I.F., Sernyak P.S. et al. Diseases of the prostate. - Zdorov'ya, Kiev. -1984. - 280 pp.
2. Tkachuk V.N., Gorbachev A.G., Agulyansky L.I. Chronic prostatitis. - Medicina, Leningrad. - 1989. - 208 pp.
3. Mashkovsky M.D. Pharmaceutical substances (Manual for Physicians). - Medicina, Moscow. - 1993. - Part I. - 736 pp. - Part II. - 688 pp.
4. Register of Pharmaceutical Substances of Russia. Edition 6. - RLS-2000, Moscow.\-1998.-1072 pp.
5. Patent of the Russian Federation No. 1417244 «Method of obtaining a substance restoring the prostate function», International Classification of Inventions (ICI) A61 K 38/00, 1993.
6. Yakubke Kh.-D., Eshkeit Kh. Amino acids, peptides, proteins: Translated from German. - Mir, Moscow. - 1985. - 456 pp.

## Claims

1. Tetrapeptide Lys-Glu-Asp-Pro.

2. Tetrapeptide Lys-Glu-Asp-Pro capable of regulating the prostate function.

3. Pharmaceutical preparation intended for the treatment for diseases and pathologic states requiring regulation of the prostate functions and **characterised by** the fact that it contains a effective amount of tretrapeptide Lys-Glu-Asp-Pro or one of its salts and a pharmaceutically admissible carrier.

4. Preparation according to Claim 3 **characterised by** the fact that it contains salts of the amino group selected from acetate, hydrochtoride or oxalate.

5. Preparation according to Claim 3 **characterised by** the fact that it contains salts of carboxyl groups selected from the salts of sodium, potassium, calcium, lithium, zinc, magnesium, ammonium or triethylammonium.

6. Preparation according to Claim 3-5 **characterised by** the fact that it is intended for parenteral administration.

7. Preparation according to Claims 3 - 6 **characterised by** the fact that it is active when administered in the doses of 0.01-100 pg/kg of the body weight.

8. Use of the tetrapeptide Lys-Glu-Asp-Pro or one of ist salts and a pharmaceutically admissible carrier for the preparation of a pharmaceutical preparation for regulating the prostate function by administering to a patient of 0.01-100 µg/kg of the body weight of said pharamaceutical preparation at least once a day during a period required for attaining a therapeutic effect.

9. Use according to claim 8 wherein the preparation is intended for parental administration.

## Patentansprüche

1. Tetrapeptid Lys-Glu-Asp-Pro.

2. Tetrapeptid Lys-Glu-Asp-Pro, das die Prostatafunktion regulieren kann.

3. Pharmazeutisches Präparat zur Behandlung von Erkrankungen und pathologischen Zuständen, die eine Regulation der Prostatafunktionen benötigen, **gekennzeichnet durch** die Tatsache, dass es eine wirksame Menge an Tetrapeptid Lys-Glu-Asp-Pro oder eines seiner Salze und einen pharmazeutisch annehmbaren Träger enthält.

4. Präparat gemäß Anspruch 3, **dadurch gekennzeichnet, dass** es Salze der Aminogruppe enthält, ausgewählt aus Acetat, Hydrochlorid oder Oxalat.

5. Präparat gemäß Anspruch 3, **dadurch gekennzeichnet, dass** es Salze der Carboxylgruppen enthält, ausgewählt aus den Salzen von Natrium, Kalium, Calcium, Lithium, Zink, Magnesium, Ammonium oder Triethylammonium.

6. Präparat gemäß einem der Ansprüche 3 bis 5, **gekennzeichnet dadurch, dass** es zur parenteralen Verabreichung vorgesehen ist.

7. Präparat gemäß Ansprüchen 3 bis 6, **dadurch gekennzeichnet, dass** es wirksam ist, wenn es in Dosierungen von 0,01 bis 100 µg/kg Körpergewicht verabreicht wird.

8. Verwendung des Tetrapeptids Lys-Glu-Asp-Pro oder eines seiner Salze und eines pharmazeutisch annehmbaren Trägers zur Herstellung eines pharmazeutischen Präparats zur Regulation der Prostatafunktion durch Verabreichen an einem Patienten von 0,01 bis 100 µg/kg Körpergewicht des pharmazeutischen Präparats mindestens einmal am Tag über einen Zeitraum notwendig, um eine therapeutische Wirkung zu erzielen.

9. Verwendung gemäß Anspruch 8, wobei das Präparat zur oralen Verabreichung vorgesehen ist.

## Revendications

1. Tétrapeptide Lys-Glu-Asp-Pro.

2. Tétrapeptide Lys-Glu-Asp-Pro capable de régler la fonction prostate.

3. Préparation pharmaceutique destinée au traitement de maladies et d'états pathologiques nécessitant de régler les fonctions prostate et **caractérisée par le fait qu'**elle contient une quantité effective de tétrapeptide Lys-Glu-Asp-Pro ou l'un de ses sels et un véhicule pharmaceutiquement acceptable.

4. Préparation selon la revendication 3, **caractérisée par le fait qu'**elle contient des sels du groupe amino choisis parmi l'acétate, le chlorhydrate ou l'oxalate.

5. Préparation selon la revendication 3, **caractérisée par le fait qu'**elle contient des sels du groupe carboxyle choisis parmi les sels de sodium, potassium, calcium, lithium, zinc, magnésium, ammonium ou triéthylammonium.

6. Préparation selon l'une des revendications 3 à 5, **caractérisée par le fait qu'**elle est destinée à une administration parentérale.

7. Préparation selon l'une des revendications 3 à 6, **caractérisée par le fait qu'**elle est active lorsqu'elle est administrée à des doses de 0,01 à 100 µg/kg du poids corporel.

8. Utilisation du tétrapeptide Lys-Glu-Asp-Pro ou l'un de ses sels et un véhicule pharmaceutiquement acceptable pour la préparation d'une préparation pharmaceutique pour régler la fonction prostate par l'administration à un patient de 0,01 à 100 µg/kg du poids corporel de ladite préparation pharmaceutique au moins une fois par jour pendant une période nécessaire pour obtenir un effet thérapeutique.

9. Utilisation selon la revendication 8, dans laquelle la préparation est destinée à une administration parentérale.
